# EUROPÄISCHE PATENTSCHRIFT

(11) **EP 1 924 305 B1**
(45) Veröffentlichungstag und Bekanntmachung des Hinweises auf die Patenterteilung: **24.02.2010**
(21) Anmeldenummer: 06777188.1
(22) Anmeldetag: 14.09.2006
(51) Int. Cl.: A61M 3/02, B65D 83/14, B05B 9/08

(54) **EINRICHTUNG ZUR ERZEUGUNG EINES FLÜSSIGKEITSSTRAHLES ZUM REINIGEN VON WUNDEN**
DEVICE FOR GENERATING A JET OF LIQUID FOR CLEANING WOUNDS
DISPOSITIF DE PRODUCTION D'UN JET DE LIQUIDE PERMETTANT LE NETTOYAGE DE PLAIES

(30) Priorität: 14.09.2005 CH 14922005
(43) Veröffentlichungstag der Anmeldung: 28.05.2008
(73) Patentinhaber: MEDAXIS AG, 5000 Aarau (CH)
(72) Erfinder: BRÄNDLI, Mark, CH-5018 Erlinsbach (CH)
(74) Vertreter: Luchs, Willi
(86) Internationale Anmeldenummer: PCT/EP2006/008938
(87) Internationale Veröffentlichungsnummer: WO 2007/031304

(56) Entgegenhaltungen:
- EP-A1- 0 338 844
- CH-A5- 685 802
- US-A- 1 356 519

## Beschreibung

Die Erfindung betrifft eine Einrichtung zur Erzeugung eines Flüssigkeitsstrahtes gemäss dem Oberbegriff des Anspruches 1.

Die Einrichtung eignet sich zum Druckspülen und Dekontaminieren von chirurgischen oder chronischen Wunden und von infizierten und/oder verunreinigten Körperstellen.

Eine Einrichtung dieser Art ist beispielsweise in der WO 00/59385 offenbart. Zur Erzeugung eines Flüssigkeitsdruckes ist ein in einem Gehäuse untergebrachtes Pumpenaggregat vorhanden, das als eine Differentialkolbenpumpe ausgebildet ist, deren Niederdruckteil an ein Druckversorgungsmittel, z.B. einen Kompressor, angeschlossen ist, während der Hochdruckteil über einen Schlauch an einen Flüssigkeitsbehälter einerseits und über eine flexible Leitung an eine den Flüssigkeitsstrahl auslassende Düse anderseits angeschlossen ist.

Die Einrichtung nach der WO 00/59385 ist massiv und schwer ausgebildet und für ambulante Behandlungen bzw. mobile Anwendungen weniger geeignet. Ausserdem wird sie für das Debridieren, also zum Entfernen von biologischem Gewebe und Fibrin- und Nekorsebelägen eingesetzt und mit wesentlich höheren Drücken betrieben. Es sind daher entsprechend aufwendigere und dementsprechend massivere Gerätschaften erforderlich.

Die Druckschrift EP-A-0 338 844 zeigt eine Vorrichtung zur Abgabe einer sterilen Flüssigkeit, die für medizinische und ähnliche Zwecke angewendet wird. Bei dieser ist ein Behälter vorgesehen, in dem eine Flüssigkeit vorteilhaft unter Druck gehalten ist und welcher einen Abgabeweg mit einem Speiserohr sowie eine Druckversorgung aufweist. Oben beim Behälter ist eine handbetätigbare Sprühdüse angeordnet. Bei einer Ausführung ist innerhalb des Behälters eine Ausnehmung enthalten, in welcher eine (bomb) mit einer Auslassdüse versehen ist, durch welche ein Gas in den Behälter zur Bildung des notwendigen Druckes erzeugt wird.

Es wird mit dieser Einrichtung ein verhältnismässig geringer Druck für das Aussprühen der Flüssigkeit erzeugt, dies insbesondere auch deshalb, weil der Druck durch diese Anordnung eines Filters zusätzlich gedrosselt wird.

Bei der Druckschrift CH-A-685 802 ist ein Spülgerät zur Behandlung von Zahnfleisch geoffenbart, bei welchem mittels eines Druckbehälters eine Spüllösung durch eine an den Behälter angeschlossene Kanüle mit einer Düse gespritzt werden kann. Zur Erzeugung des Druckes in dem Behälter ist ein von Hand betätigbarer Pumpkolben integriert, mittels dem wiederum ein relativ geringer Druck auf die Spüllösung erzeugt werden kann.

Gemäss der Druckschrift US-A-1,356,519 ist ein tragbares Gerät geoffenbart, welches für das Dispensieren einer antiseptischen Lösung geeignet ist, mittels dem infizierte Wunden behandelt werden. Es ist hierbei ein erster Behälter, in welchem ausreichend Flüssigkeit enthalten sein soll, und ein mit diesem über einen verschliessbaren Schlauch verbundener zweiter Behälter vorgesehen. Von letzterem kann via eine Schlauchverbindung und eine auswechselbar Düse die Flüssigkeit gesprüht werden.

Der vorliegenden Erfindung liegt die Aufgabe zugrunde, eine Einrichtung der eingangs genannten Art zu schaffen, die problemlos transportierbar ist und dadurch nicht nur für stationären Betrieb, sondern auch für ambulante Behandlungen bzw. mobile Anwendungen geeignet ist.

Diese Aufgabe wird erfindungsgemäss durch eine Einrichtung mit den Merkmalen des Anspruches 1 gelöst.

Weitere bevorzugte Ausgestaltungen der erfindungsgemässen Einrichtung bilden den Gegenstand der abhängigen Ansprüche.

Dadurch, dass erfindungsgemäss der Flüssigkeitsbehälter als ein über ein Ventil an eine Gaspatrone oder einen Druckluftanschluss anschliessbarer Druckbehälter ausgebildet ist, an den die flexible, zu der Düse führende Leitung anschliessbar ist, wobei der Flüssigkeitsbehälter über ein Absperr- bzw. Regulierorgan mit der Leitung verbindbar ist, wird eine einfache, mühelos transportierbare und auch problemlos sterilisierbare Einrichtung geschaffen, die auch für ambulante Behandlungen geeignet ist und nicht nur von Ärzten, sonder auch vom Pflegepersonal verwendet werden kann, z.B. für die Krankenpflege zuhause (Spitex etc.). Die erfindungsgemässe Einrichtung ist insbesondere zu einer Druckspülung und Dekontamination von chirurgischen und chronischen Wunden und zur Spülung von infizierten oder verunreinigten Körperstellen besonders geeignet.

Die Erfindung wird nachfolgend anhand der Zeichnung näher erläutert. Es zeigen rein schematisch:
- Fig. 1: ein Ausführungsbeispiel einer erfindungsgemässen Einrichtung zur Erzeugung eines Flüssigkeitsstrahles in Seitenansicht; und
- Fig. 2: eine am Ende einer flexiblen Leitung angeordnete Düse.

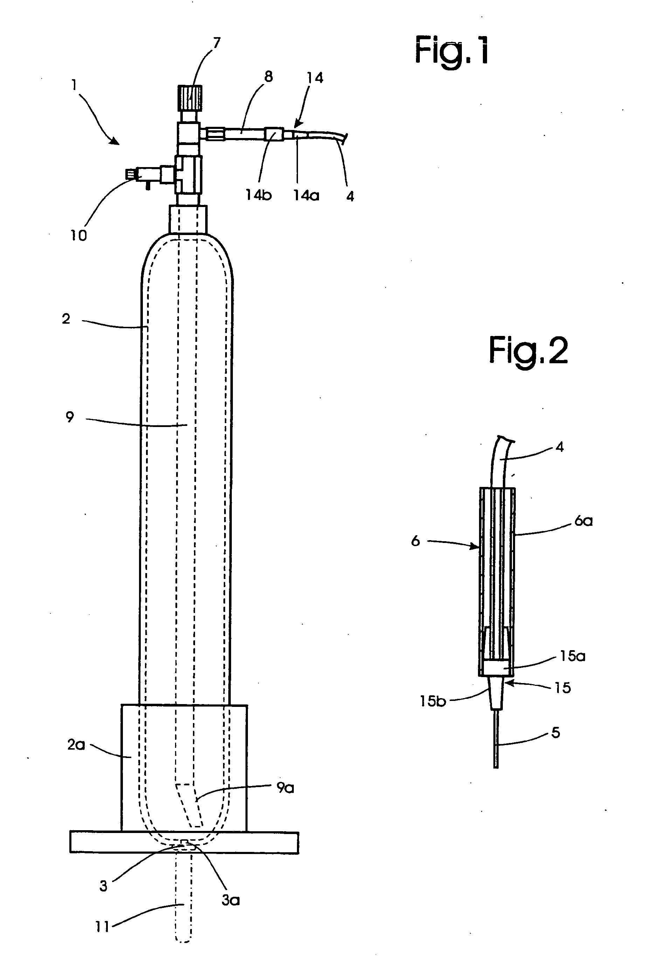

Fig.1 zeigt eine Einrichtung 1 zur Erzeugung eines Flüssigkeitsstrahles, für die Druckspülung und Dekontamination von chirurgischen oder chronischen Wunden oder zur Druckspülung von infizierten oder verunreinigten Körperstellen oder ähnlichem.

Die Einrichtung 1 weist einen Flüssigkeitsbehälter 2 mit einem Ständer 2a auf, der erfindungsgemäss als ein vorzugsweise flaschenförmiges Druckbehälter ausgebildet ist, und der über ein in seinem Bodenbereich angeordnetes Ventil 3 an eine Druckluftquelle angeschlossen werden kann, um einen Flüssigkeitsdruck zu erzeugen. Bei einem mobilen Betrieb der Einrichtung 1 kann als Druckluftquelle eine Gaspatrone 11 eingesetzt werden, bei stationärem Betrieb kann die Druckluft direkt von einem Druckluft-Netzanschluss im Spital eingespiesen werden.

Im Flüssigkeitsbehälter 2 ist eine physiologische Lösung bzw. ein Antiseptikum enthalten, beispielsweise eine sterile Kochsalzlösung oder dergleichen, die unter Druck über eine an den Flüssigkeitsbehälter 2 angeschlossene flexible Leitung 4 zu einer am Ende der Leitung 4 befestigten und den Flüssigkeitsstrahl erzeugenden Düse 5 geleitet werden kann. Die Düse 5, die aus Fig. 2 ersichtlich ist und weiter unten noch näher beschrieben wird, bildet einen Teil eines mit einem Handgriff 6a versehenen Handstückes 6, das für manuelle Handhabung vorgesehen ist.

Im oberen Bereich der Einrichtung 1, oberhalb des Flüssigkeitsbehälters 2, ist ein Absperr- bzw. Regulierorgan, vorzugsweise ein Regulierhahn 7, angeordnet; mit welchem die aus dem Flüssigkeitsbehälter 2 über ein Anschlussstück 8 in die flexible Leitung 4 geführte Flüssigkeitsmenge eingestellt werden kann. Im Innern des Flüssigkeitsbehälters 2 befindet sich ein Tauchrohr 9, das über den Regulierhahn 7 mit dem Anschlussstück 8 verbunden ist und sich vom Regulierhahn 7 nach unten, zum Behälterboden hin, bis oberhalb von der Gaseinfüllöffnung 3a, erstreckt. Mit Vorteil ist das Tauchrohr 9 am unteren Ende 9a leicht abgewinkelt, damit beim Gaseinfüllen möglichst wenig Gas ins Tauchrohr 9 fliesst und so verloren geht. Die Einrichtung 1 ist ferner mit einem Entlüfter, vorzugsweise einem regelbaren Entlüftungsventil (oder Entlüftungshahn) 10, ausgestattet, mittels welchem bei Bedarf Luft/Gas abgesaugt werden kann, damit der Flüssigkeitsbehälter 2 schneller befüllt werden kann.

Die mit der Flüssigkeit in Kontakt kommenden Teile der Einrichtung 1 bzw. des Flüssigkeitsbehälters 2 inklusive sämtliche Armaturen werden vorzugsweise aus Edelstahl angefertigt und lassen sich sowohl mit Dampf wie auch im Plasmasterilisator sterilisieren. Auch die flexible Leitung 4 kann aus einem geeigneten Metall bestehen, ist aber vorzugsweise in Kunststoff gefertigt.

Die flexible Leitung 4 wird vorzugsweise mit einem Normanschluss, z.B. mit einem sogenannten Luer-Lock-Anschluss 14 mit dem diesem Regulierhahn 7 nachgeschalteten Anschlussstück 8 verbunden, der einen leitungsfesten Teil 14a sowie einen dem Anschlussstück 8 zugeordneten Teil 14b (einer Überwurfmutter) umfasst.

Auch das am anderen Ende der flexible Leitung 4 angeordnete und die Düse 5 aufweisende Handstück 6 ist mit Vorteil über einen Normanschluss wie z.B. einen Luer-Lock-Anschluss 15 an die Leitung 4 angeschlossen, derart, dass ein Ausschlüpfen unter Druck nicht möglich ist. Der Leitung 4 ist ein Anschlussteil 15a (eine Überwurfmutter) zugeordnet, die Düse 5, die erfindungsgemäss als eine Einweg-Nadel bzw. Kanüle aus Kunststoff oder Metall ausgebildet ist, ist mit einem Anschlussteil 15b verbunden und zusammen mit diesem nach dem Gebrauch wegwerfbar. Es können aber auch Handstückanschlüsse, die nicht einer Norm entsprechen, leicht adaptiert werden, indem nur die Überwurfmutter gelöst wird und eine angepasster Adapter angebracht wird.

Am Anschlussteil 15a ist der bereits erwähnte Handgriff 6a angebracht, vorzugsweise aufgeklebt, der rohrförmig ausgebildet und beispielsweise aus Kunststoff oder Karton gefertigt ist.

Die flexible Leitung 4 sowie die ihr zugeordneten und mit dieser z.B. vergossenen Anschlussteile 14a, 15a sowie auch die Nadel können - der Fig. 2 entsprechend - beispielsweise aus Kunststoff bestehen.

Die Materialien der einzelnen Teile der Einrichtung 1 sind so gewählt, dass diese gegen Hitze, Dampf und alle Arten von Desinfektionsmitteln resistent sind, damit die metallischen Teile mit Heissdampf autoklaviert oder mit Instrumenten-Desinfektionsmitteln desinfiziert werden können. Die Einwegteile, die vorzugsweise aus Kunststoff gefertigt sind, werden plasmasterilisiert oder können mit Gammastrahlen sterilisiert werden.

Die erfindungsgemässe Einrichtung zur Erzeugung eines Flüssigkeitsstrahles für die Druckspülung und Dekontamination von chirurgischen oder chronischen Wunden und von infizierten Körperstellen oder ähnlichem ist herstellungs- und montagetechnisch einfach und kostengünstig. Sie ist problemlos transportierbar und dadurch nicht nur für stationären Betrieb, sondern auch für ambulante Behandlungen bzw. mobile Anwendungen geeignet. Verwendet werden kann sie nicht nur von Ärzten, sondern auch vom Pflegepersonal, z.B. für die Krankenpflege zuhause (Spitex etc.).

## Patentansprüche

1. Einrichtung zur Erzeugung eines Flüssigkeitsstrahles zur Behandlung von Wunden oder infizierten Körperstellen, mit einem Flüssigkeitsbehälter (2), mit Mitteln zur Erzeugung eines Flüssigkeitsdruckes und mit einer am Ende einer flexiblen Leitung (4) angeordneten, den Flüssigkeitsstrahl auslassenden Düse (5),
**dadurch gekennzeichnet, dass**
der Flüssigkeitsbehälter (2) als ein über ein Ventil (3) an eine Gaspatrone oder einen Druckluftanschluss anschliessbarer Druckbehälter ausgebildet ist, an den die flexible, zu der Düse (5) führende Leitung (4) anschliessbar ist, wobei der Flüssigkeitsbehälter (2) über ein als ein Regulierhahn ausgebildetes Absperr- und Regulierorgan (7) mit der Leitung (4) verbindbar ist, wobei ein Entlüftet (10) zum Absaugen oder freien Abfliessen lassen von Gas/Luft aus dem Flüssigkeitsbehälter (2) vorhanden ist, der als ein regelbares Entlüftungsventil oder ein Entlüftungshahn ausgebildet ist, und dass sich die Einrichtung (1) für die Druckspülung und Dekontamination von chirurgischen oder chronischen Wunden oder von infizierten Körperstellen oder ähnlichem eignet.

2. Einrichtung nach Anspruch 1, **dadurch gekennzeichnet, dass** innerhalb des Flüssigkeitsbehälters (2) ein Tauchrohr (9) vorgesehen ist, das über das Absperr- bzw. Regulierorgan (7) mit einem Anschlussstück (8) für die flexible Leitung (4) verbunden ist.

3. Einrichtung nach Anspruch 2, **dadurch gekennzeichnet, dass** sich das Tauchrohr (9) vom Absperr- bzw. Regulierorgan (7) nach unten, zum Behälterboden hin, bis oberhalb von einer Gaseinfüllöffnung (3a), erstreckt und am unteren Ende (9a) vorzugsweise leicht abgewinkelt ist.

4. Düse für eine Einrichtung nach einem der Ansprüche 1 bis 3, **dadurch gekennzeichnet, dass** die Düse (5) eine Einweg-Nadel bzw. Kanüle mit einer nadelförmigen Ausbildung aufweist, die über einen Anschluss (15) am Ende der flexiblen Leitung (4) angeordnet ist.

5. Düse nach Anspruch 4, **dadurch gekennzeichnet, dass** auf einen mit dem Ende der flexiblen Leitung (4) fest verbundenen Anschlussteil (15a) des Anschlusses (15) ein rohrförmiger Handgriff (6a) aufgesetzt und mit diesem fest verbunden ist.

6. Düse nach Anspruch 4 oder 5, **dadurch gekennzeichnet, dass** die Einweg-Nadel bzw. Kanüle mit einer nadelförmigen Ausbildung aus Kunststoff oder Metall besteht.

## Claims

1. Device to generate a jet of liquid for the treatment of wounds or infected areas of the body, with a liquid container (2), with means for generating a liquid pressure, and with a nozzle (5), which is arranged at the end of a flexible conduit (4) and from which the jet of liquid emerges, **characterised in that**
the liquid container (2) is designed as a pressurised container, which can be connected via a valve (3) to a gas cartridge or a compressed-air attachment, to which the flexible conduit (4) leading to the nozzle (5) can be joined, whereby the liquid container (2) can be connected to the conduit (4) via a shut-off - and control member (7) in the form of a regulating tap, whereby a drainage means (10) to suction out or allow gas/air to flow freely out of the liquid container (2) is provided, which is in the form of an adjustable drain valve or a drain tap, and that the device (1) is suitable for the pressurised irrigation and decontamination of surgical or chronic wounds or of infected areas of the body or the like.

2. Device according to claim 1, **characterised in that** an immersion pipe (9) is provided inside the liquid container (2), which being connected via the shut-off- respective control member (7) with a connection piece (8) for the flexible conduit (4).

3. Device according to claim 2, **characterised in that** the immersion pipe (9) of the shut-off respective control member (7) extends downwards towards the base of the container, to above a gas fill opening (3a) and is preferably slightly angled at the lower end (9a).

4. Nozzle for a device according to one of the claims 1 to 3, **characterised in that** the nozzle (5) has a disposable needle or cannula with a needle-shaped design, which is arranged on the end of the flexible conduit (4) via a connector (15).

5. Nozzle according to claim 4, **characterised in that** a tubular handle (6a) is positioned on a connection piece (15a) of the connector (15), which is securely attached to the end of the flexible conduit (4) and is securely attached hereto.

6. Nozzle according to claim 4 or 5, **characterised in that** the disposable needle respective cannula with a needle-shaped design is made of plastic or metal.

## Revendications

1. Dispositif pour produire un jet de liquide destiné au traitement de plaies ou de parties du corps infectées, comportant un réservoir de liquide (2), des moyens pour produire une pression de liquide et une tuyère (5) disposée à l'extrémité d'une conduite flexible (4) qui laisse sortir le jet de liquide, **caractérisé en ce que**
le réservoir de liquide (2) est conçu sous forme de réservoir sous pression pouvant être raccordé à une cartouche de gaz ou à une prise d'air comprimé par l'intermédiaire d'une soupape (3), sur lequel la conduite flexible (4), conduisant à la tuyère (5), peut être raccordée, le réservoir de liquide (2) pouvant être relié à la conduite (4) par l'intermédiaire d'un organe de sectionnement et de régulation (7), en forme de robinet de régulation, un purgeur (10) étant présent pour aspirer ou laisser s'écouler librement le gaz/l'air depuis le réservoir de liquide (2), conçu sous forme de soupape de purge ou de purgeur d'air réglable, et **en ce que** le dispositif (1) convient pour le nettoyage sous pression et la décontamination de plaies chirurgicales ou chroniques ou de parties du corps infectées ou autres.

2. Dispositif selon la revendication 1, **caractérisé en ce qu'**à l'intérieur du réservoir de liquide (2) se trouve un tube plongeur (9) qui est relié à un raccord (8) de la conduite flexible (4) par l'intermédiaire de l'organe de sectionnement et/ou de régulation (7).

3. Dispositif selon la revendication 2, **caractérisé en ce que** le tube plongeur (9) est dirigé vers le bas depuis l'organe de sectionnement et/ou de régulation (7), vers le fond du réservoir, jusqu'au-dessus d'une ouverture de remplissage de gaz (3a) et de préférence, légèrement coudé sur l'extrémité inférieure (9a).

4. Tuyère pour un dispositif selon l'une quelconque des revendications 1 à 3, **caractérisée en ce que** la tuyère (5) est une aiguille à usage unique ou une canule en forme d'aiguille, disposée sur l'extrémité de la conduite flexible (4) par l'intermédiaire d'un raccord (5).

5. Tuyère selon la revendication 4, **caractérisée en ce qu'**une poignée tubulaire (6a) est posée sur une partie de raccord (15a) du raccord (15) reliée fixement à l'extrémité de la conduite flexible (4) et reliée solidement à celle-ci,

6. Tuyère selon la revendication 4 ou la revendication 5, **caractérisée en ce que** l'aiguille à usage unique ou la canule en forme d'aiguille est en matière plastique ou en métal.
